# EUROPEAN PATENT APPLICATION

(11) **EP 4 442 660 A1**
(43) Date of publication of application: **09.10.2024**
(21) Application number: 22913285.7
(22) Date of filing: 08.07.2022
(51) Int. Cl.: C03C 10/04, C03C 12/00, C03C 1/04, A61K 6/882, A61K 6/818, A61K 6/811, A61K 6/78, C04B 37/00, C04B 35/14, C04B 41/85

(54) **CERAMIC MATERIAL AND REPAIR MATERIAL FOR IMPROVING SURFACE ADHESION OF DENTAL ZIRCONIA, AND PREPARATION METHOD AND BONDING METHOD FOR REPAIR MATERIAL**

(30) Priority: 30.12.2021 CN 202111648654
(71) Applicant: Aidite (Qinhuangdao) Technology Co., Ltd., Qinhuangdao Hebei 066004 (CN)
(72) Inventor: ZHANG, Jiaxin, Qinhuangdao, Hebei 066004 (CN); NIE, Quanyi, Qinhuangdao, Hebei 066004 (CN); ZHENG, Jingjing, Qinhuangdao, Hebei 066004 (CN); WANG, Zhe, Qinhuangdao, Hebei 066004 (CN); WEI, Linlin, Qinhuangdao, Hebei 066004 (CN)
(74) Representative: Cicconetti, Andrea
(86) International application number: PCT/CN2022/104480
(87) International publication number: WO 2023/123996

(57) **Abstract**

A ceramic material for improving surface adhesion of dental zirconia, a restorative material and a preparation method and a pasting method thereof are provided. The ceramic material includes SiO₂, B₂O₃, GeO₂, Al₂O₃, Li₂O, Nb₂O₅, Na₂O, ZrO₂, P₂O₅, and a rare earth oxide. The ceramic material contains GeO₂, on one hand, GeO₂ can replace SiO₂ in the ceramic material, thereby reducing the high-temperature melting temperature of glass, and saving the production cost, and on the other hand, GeO₂ can effectively reduce the ratio of Si to Li, such that fine and uniform lithium disilicate crystals are formed after crystallization, and the adhesion strength with a zirconium oxide matrix is improved.

## Description

### CROSS REFERENCE TO RELATED APPLICATION

This patent application claims the benefit and priority of Chinese Patent Application No. 202111648654.1, entitled "Ceramic material for improving surface adhesion of dental zirconia, restorative material and preparation method and pasting method thereof filed on December 30, 2021, the disclosure of which is incorporated by reference herein in its entirety as part of the present application.

### TECHNICAL FIELD

The present application belongs to the technical field of dental materials, and specifically relates to a ceramic material for improving surface adhesion of dental zirconia, a restorative material and a preparation method and a pasting method thereof.

### BACKGROUND ART

With the development of computer-aided design and computer-aided manufacturing (CAD/CAM) technology, all-ceramic restorative materials have gradually become one of the main restorative materials selected by patients due to their high strength, desired biological properties and excellent aesthetic effect. The all-ceramic restorative materials mainly include yttria-stabilized zirconia (Y-TZP), lithium disilicate glass ceramics, feldspar glass ceramics, and white garnet glass ceramics. Among them, zirconia is widely used in dental veneers, inlays, onlays, single crowns, and three-unit pontic and multi-unit pontic due to its ultra-high flexural strength (>800 Mpa) and fracture toughness (>5 Mpa·m^{1/2}). However, compared with other all-ceramic restorative materials, zirconia is difficult to be etched by HF due to its dense crystal structure. As a result, it is difficult for zirconia to achieve desired adhesion properties with an abutment tooth, limiting the use of zirconia in veneer restorations to a large extent. Therefore, a large number of existing technologies are devoted to improving the adhesion properties of zirconia through various methods that can be roughly divided into two categories: physical treatment of zirconia surface and surface coating.

The physical treatment method is mainly to increase the roughness of the zirconia surface by alumina sandblasting, thus improving the mechanical interlocking of zirconia with adhesion agents. However, alumina sandblasting is easy to cause the irregular surface morphology of zirconia, easy to generate microcracks, and induces a tetragonal phase to a monoclinic phase, resulting in a decrease in the adhesion strength and stability of zirconia.

CN110590401A discloses a method for surface treating a sintered zirconia material and use thereof. The surface treatment of sintered zirconia is conducted by ultra-short pulse laser to increase the surface roughness of zirconia and improve the adhesive strength between its surface and an adhesive. However, the surface treated by ultra-short pulse laser is generally linear. This retention structure can easily lead to the dissolution of the adhesive, thereby easily leading to a decrease in the adhesion strength during aging.

CN103431918A discloses a novel method for surface treating a dental zirconia-based ceramic and improving adhesion properties thereof. By means of thermal acid etching, the zirconia ceramic is immersed in an H₂SO₄/(NH₄) ₂SO₄ acid mixture solution at 100°C for 20-30 min to improve the roughness of the zirconia surface. However, this thermal acid etching method is difficult to control the range of acid etching. In actual operations, the strong acid volatilized at high temperatures will have a negative impact on the environment and human health.

Surface coating is an environmental-friendly processing technique that does not destroy the surface roughness of zirconia. The surface coating is mainly conducted by coating or spraying a layer of decorative glass-ceramics on the surface of zirconia to meet the aesthetic requirements of dental restorations or to solve the adhesion problems of zirconia.

CN104774007A discloses a functionally-graded zirconia ceramic material partially infiltrated by a dental glass. By infiltrating a layer of glass-ceramics on a surface of pre-sintered porous zirconia, a veneer glass ceramic combined with three layers of glass, glass-infiltrated zirconia, and dense zirconia is formed to improve the adhesion strength of the veneering porcelain and zirconia.

CN102432337 discloses a surface treatment method of a zirconia body. A glass-ceramic layer is fused on a surface of zirconia. Then the glass-ceramic layer is completely etched by HF to form a rough zirconia surface, thereby improving the adhesion strength of veneering porcelain and zirconia.

CN103395982 discloses a ceramic veneer containing Y-TZP for a dental restoration. By controlling the formula of veneering porcelain, a veneer glass ceramic with lithium disilicate as the main crystalline phase is formed to achieve a desired aesthetic effect and adhesion strength.

However, although the above methods provide various ideas for the adhesion of zirconia, they do not actually solve the difficulty of zirconia adhering to veneer restorations. Therefore, it is a technical problem to be solved urgently at present to develop a material with new technology, simple process operation and environment-friendly material to solve the adhesion of zirconia.

### SUMMARY

An objective of the present application is to provide a ceramic material for improving surface adhesion of dental zirconia, a restorative material and a preparation method and a pasting method thereof. In the present application, the ceramic material comprises GeO₂. On the one hand, GeO₂ can replace SiO₂ in ceramic materials, reducing the high melting temperature of glasses, and saving production costs. On the other hand, GeO₂ can effectively reduce a ratio of Si to Li so that fine and uniform lithium disilicate crystals can be formed after crystallization, thereby improving the adhesion strength between the lithium disilicate crystal and a zirconia substrate.

In order to achieve the above objective, the present application provides the following technical solutions:

In the first aspect, the present application provides a ceramic material for improving surface adhesion of dental zirconia, comprising SiO₂, B₂O₃, GeO₂, Al₂O₃, Li₂O, Nb₂O₅, Na₂O, ZrO₂, P₂O₅, and a rare earth oxide.

In the present application, the ceramic material comprises GeO₂. On the one hand, GeO₂ can replace SiO₂ in ceramic materials, reducing the high melting temperature of glasses, and saving production costs. On the other hand, GeO₂ can effectively reduce a ratio of Si to Li so that fine and uniform lithium disilicate crystals can be formed after crystallization, thereby improving the adhesion strength between the lithium disilicate crystal and a zirconia substrate.

In some embodiments, based on the mass fraction of the ceramic material as 100 wt%, the ceramic material comprises the following components by mass fraction:

| | |
|---|---|
| SiO₂ | 54-68 wt% |
| B₂O₃ | 2-6 wt% |
| GeO₂ | 1-10 wt% |
| Al₂O₃ | 0.2-4 wt% |
| Li₂O | 20-40 wt% |
| Nb₂O₅ | 2-10 wt% |
| Na₂O | 2-10 wt% |
| ZrO₂ | 0.2-5 wt% |
| P₂O₅ | 0.2-8 wt% |
| a rare earth oxide | 0-4 wt% |

For example, SiO₂ has a mass fraction of 54 wt%, 57 wt%, 59 wt%, 63 wt%, 65 wt% or 68 wt%; B₂O₃ has a mass fraction of 2 wt%, 3 wt%, 4 wt%, 5 wt% or 6 wt%; GeO₂ has a mass fraction of 1 wt%, 3 wt%, 5 wt%, 7 wt%, 9 wt% or 10 wt%; Al₂O₃ has a mass fraction of 0.2 wt%, 0.5 wt%, 1 wt%, 2 wt%, 3 wt% or 4 wt%; Li₂O has a mass fraction of 20 wt%, 25 wt%, 30 wt%, 35 wt% or 40 wt%; Nb₂O₅ has a mass fraction of 2 wt%, 4 wt%, 6 wt%, 8 wt% or 10 wt%; Na₂O has a mass fraction of 2 wt%, 4 wt%, 6 wt%, 8 wt% or 10 wt%; ZrO₂ has a mass fraction of 0.2 wt%, 0.5 wt%, 1 wt%, 3 wt% or 5 wt%; P₂O₅ has a mass fraction of 0.2 wt%, 0.5 wt%, 1 wt%, 3 wt%, 6 wt% or 8 wt%; the rare earth oxide has a mass fraction of 0, 0.5 wt%, 1 wt%, 2 wt%, 3 wt% or 4 wt%. However, the numerical values are not limited to the listed values, and the other unlisted values within the respective numerical ranges are also applicable.

It should be noted that, in the present application, the mass fraction of GeO₂ is limited to 1 wt% to 10 wt%. Too high or too low mass fraction of GeO₂ may affect the adhesion strength of the final restorative material. This is because when the content of GeO₂ is lower than 1 wt%, the amount of GeO₂ to substitute SiO₂ in the glass-ceramics will be decreased, resulting in the uneven melting of glass-ceramics below 1,350°C and serious phase separation, thereby leading to a decrease in adhesion strength of the restorations; when the content of GeO₂ is higher than 10 wt%, the ratio of Si to Li may be significantly reduced, resulting in the lithium disilicate crystal formed by crystallization being very small, and the crystals cannot achieve effective cross-linking and interlocking so that the adhesion strength of the restorations cannot be improved.

In some embodiments, based on the mass fraction of the ceramic material as 100 wt%, the ceramic material comprises the following components by mass fraction:

| | |
|---|---|
| SiO₂ | 55-65 wt% |
| B₂O₃ | 3-5 wt% |
| GeO₂ | 2-6 wt% |
| Al₂O₃ | 0.8-3 wt% |
| Li₂O | 20-40 wt% |
| Nb₂O₅ | 3-8 wt% |
| Na₂O | 3-6 wt% |
| ZrO₂ | 0.2-3 wt% |
| P₂O₅ | 0.4-6 wt% |
| a rare earth oxide | 0.4-3 wt%. |

In some embodiments, the rare earth oxide comprises any one or a combination of at least two selected from the group consisting of Nd₂O₃, Tb₂O₃, Pr₆O₁₁, La₂O₃, Eu₂O₃, and Er₂O₃, and the typical but non-restrictive examples of the combination comprise a combination of Nd₂O₃ and Pr₆O₁₁, a combination of Tb₂O₃, La₂O₃ and Eu₂O₃ or a combination of Er₂O₃, Tb₂O₃, Pr₆O₁₁ and La₂O₃.

In the second aspect, the present application further provides a restorative material for improving surface adhesion of dental zirconia, comprising a zirconia substrate and a ceramic layer on a surface of the zirconia substrate, where at an interface between the ceramic layer and the zirconia substrate, lithium disilicate in the ceramic layer infiltrates into the surface of the zirconia substrate; and
the ceramic layer is prepared by the ceramic material described in the first aspect.

In some embodiments, the zirconia substrate comprises any one selected from the group consisting of dental veneers, inlays, onlays, abutment teeth, single crowns, anterior multi-unit pontic and posterior multi-unit pontica.

In the third aspect, the present application provides a method for preparing the restorative material described in the second aspect, comprising:
(I) mixing components in a ceramic material in proportion, followed by melting, quenching with water to obtain a glass slag, and subjecting the glass slag to a nucleation heat treatment and a crystallization heat treatment in sequence to obtain a lithium metasilicate glass ceramic;
(II) grinding the lithium metasilicate glass ceramic to obtain a glass powder and mixing the glass powder, a coloring material and a solvent to obtain a slurry; and
(III) spraying the slurry on an inner surface of a zirconia substrate and conducting a heat treatment so that lithium metasilicate crystals in the slurry are transformed into a lithium disilicate main crystalline phase and infiltrate into a surface of the zirconia substrate.

In the present application, in step (I), in the melting stage, it is necessary to ensure that the melting is sufficiently uniform, the water-quenched slag has no obvious phase separation phenomenon, and impurities cannot be introduced into the water-quenched slag to avoid affecting various properties and adhesion effect of the later restorative materials. The nucleation heat treatment and crystallization heat treatment are extremely important for the infiltration of lithium disilicate crystals into the inner surface of zirconia. Specifically, in the nucleation stage, the maximum nucleation rate and the optimal nucleation amount of the restorative material can be adjusted, thereby increasing the content of the lithium disilicate crystal, forming a strong interlocking force in the zirconia infiltrated layer, and improving the interface adhesion between the ceramic layer and zirconia. In step (II), through a "post-coloring" method, after the melting in step (I), the coloring material is added to the glass powder for a heat treatment to avoid the problems of easy denaturation, decomposition and volatilization of a colorant during high-temperature melting. In step (III), after the heat treatment, the slurry can infiltrate into the inner surface of the zirconia during the heat treatment to form an interface layer where the zirconia interacts with the lithium disilicate crystal, thereby improving the adhesion strength between the ceramic layer and zirconia.

In some embodiments, in step (I), the melting is conducted in a crucible.

In some embodiments, the melting is conducted at a temperature within a range of 1,050-1,350°C, such as 1,050°C, 1,100°C, 1,150°C, 1,200°C, 1,250°C, 1,300°C or 1,350°C. However, the numerical values are not limited to the listed values, and the other unlisted values within the respective numerical ranges are also applicable.

In some embodiments, the melting is conducted for 1-12 h, such as 1 h, 2 h, 3 h, 4 h, 5 h, 6 h, 7 h, 8 h, 9 h, 10 h, 11 h or 12 h. However, the numerical values are not limited to the listed values, and the other unlisted values within the respective numerical ranges are also applicable.

In some embodiments, the nucleation heat treatment is conducted at a temperature within a range of 460-580°C, such as 460°C, 470°C, 480°C, 490°C, 500°C, 510°C, 520°C, 530°C, 540°C, 550°C, 560°C, 570°C or 580°C. However, the numerical values are not limited to the listed values, and the other unlisted values within the respective numerical ranges are also applicable.

In some embodiments, the nucleation heat treatment is conducted for 30-300 min, such as 30 min, 50 min, 80 min, 100 min, 120 min, 140 min, 160 min, 180 min, 200 min, 220 min, 240 min, 260 min, 280 min or 300 min. However, the numerical values are not limited to the listed values, and the other unlisted values within the respective numerical ranges are also applicable.

In some embodiments, the crystallization heat treatment is conducted at a temperature within a range of 600-780°C, such as 600°C, 610°C, 620°C, 630°C, 640°C, 650°C, 660°C, 670°C, 680°C, 690°C, 700°C, 710°C, 720°C, 730°C, 740°C, 750°C, 760°C, 770°C or 780°C. However, the numerical values are not limited to the listed values, and the other unlisted values within the respective numerical ranges are also applicable.

In some embodiments, the crystallization heat treatment is conducted for 30-300 min, such as 30 min, 50 min, 80 min, 100 min, 120 min, 140 min, 160 min, 180 min, 200 min, 220 min, 240 min, 260 min, 280 min or 300 min. However, the numerical values are not limited to the listed values, and the other unlisted values within the respective numerical ranges are also applicable.

In some embodiments, in step (II), the glass powder has a particle size of 0.5-20 µm, such as 0.5 µm, 1 µm, 2 µm, 4 µm, 6 µm, 8 µm, 10 µm, 12 µm, 14 µm, 16 µm, 18 µm or 20 µm. However, the numerical values are not limited to the listed values, and the other unlisted values within the respective numerical ranges are also applicable.

In some embodiments, the coloring material comprises a colorant and/or a pigment.

In some embodiments, the colorant comprises any one or a combination of at least two selected from the group consisting of TiO₂, CeO₂, CuO, Cr₂O₃, MnO₂, SeO₂, V₂O₅, Pr₆O₁₁, Tb₄O₇, In₂O₃, and TaO₂, and the typical but non-restrictive examples of the combination comprise a combination of TiO₂ and CeO₂ or a combination of MnO₂, SeO₂ and V₂O₅.

In some embodiments, the pigment comprises any one or a combination of at least two selected from the group consisting of zirconium praseodymium yellow, zirconium iron red, zirconium vanadium blue, and nickel black, and the typical but non-restrictive examples of the combination comprise a combination of zirconium praseodymium yellow and zirconium iron red, a combination of zirconium vanadium blue and nickel black or a combination of zirconium praseodymium yellow, zirconium iron red and nickel black.

In some embodiments, the solvent comprises water and/or an organic solvent.

In some embodiments, in step (III), the slurry has a spraying thickness of 5-200 µm, such as 5 µm, 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, 160 µm, 170 µm, 180 µm, 190 µm or 200 µm. However, the numerical values are not limited to the listed values, and the other unlisted values within the respective numerical ranges are also applicable, preferably 20-50 µm.

In some embodiments, the heat treatment is conducted at a temperature within a range of 820-900°C, such as 820°C, 840°C, 860°C, 880°C, 890°C or 900°C. However, the numerical values are not limited to the listed values, and the other unlisted values within the respective numerical ranges are also applicable, preferably 850-890°C.

In the present application, the heat treatment is specifically limited to be conducted at a temperature within a range of 820-900°C. Too high or too low heat treatment temperature may affect the adhesion strength of the final restorative material. This is because when the heat treatment temperature is lower than 820°C, it is difficult for the lithium metasilicate crystals in the slurry to fully react to form lithium disilicate crystals, and there are still a large number of lithium metasilicate crystals that are easy to be etched by HF. As a result, during the acid etching, it is easy to leave too many pits, leading to the extremely easy formation of pores between the adhesion agent and the pits, further resulting in a decreased final adhesion strength; when the heat treatment temperature is higher than 900°C, the crystallized lithium disilicate crystals may be re-melted to form glass, failing to achieve a high adhesion strength with an adhesive.

In the fourth aspect, the present application provides a method for pasting the restorative material described in the second aspect, comprising:
acid etching the restorative material to form an acid-etched surface, coating the acid-etched surface with an adhesive, and pasting the restorative material on a surface of an abutment tooth through the adhesive.

In the present application, the restorative material needs to be acid-etched in use, followed by pasted on a surface of an abutment tooth through an adhesive. The interlocked lithium disilicate crystals can be exposed by acid etching the outer surface of the restorative material, and the mechanical interlocking and the chemical bonding reaction between the adhesive and the restorative material can be improved so that the difficult adhesion of zirconia can be effectively avoided through this transitional restorative material.

Compared with the prior art, the present application has the following beneficial effects:
(1) In the present application, the ceramic material comprises GeO₂. On the one hand, GeO₂ can replace SiO₂ in ceramic materials, reducing the high melting temperature of glasses, and saving production costs. On the other hand, GeO₂ can effectively reduce a ratio of Si to Li so that fine and uniform lithium disilicate crystals can be formed after crystallization, thereby improving the adhesion strength between the lithium disilicate crystal and a zirconia substrate.
(2) In the present application, in step (I), in the melting stage, it is necessary to ensure that the melting is sufficiently uniform, the water-quenched slag has no obvious phase separation phenomenon, and impurities cannot be introduced into the water-quenched slag to avoid affecting various properties and adhesion effect of the later restorative materials. The nucleation heat treatment and crystallization heat treatment are extremely important for the infiltration of lithium disilicate crystals into the inner surface of zirconia. Specifically, in the nucleation stage, the maximum nucleation rate and the optimal nucleation amount of the restorative material can be adjusted, thereby increasing the content of the lithium disilicate crystal, forming a strong interlocking force in the zirconia infiltrated layer, and improving the interface adhesion between the ceramic layer and zirconia. In step (II), through a "post-coloring" method, after the melting in step (I), the coloring material is added to the glass powder for a heat treatment to avoid the problems of easy denaturation, decomposition and volatilization of a colorant during high-temperature melting. In step (III), after the heat treatment, the slurry can infiltrate into the inner surface of the zirconia during the heat treatment to form an interface layer where the zirconia interacts with the lithium disilicate crystal, thereby improving the adhesion strength between the ceramic layer and zirconia.

### BRIEF DESCRIPTION OF THE DRAWINGS

FIG. 1 shows an X-ray diffraction (XRD) pattern of the ceramic material provided in Example 1 of the present application.
FIG. 2 shows a backscattered electron diagram at the interface between the ceramic layer and the zirconia substrate provided in Example 1 of the present application.
FIG. 3 shows a numerical comparison diagram of the adhesion strength between the lithium disilicate glass ceramic and the adhesion agent, and the adhesion strength between the restorative material and the adhesive provided in Example.

### DETAILED DESCRIPTION OF THE EMBODIMENTS

The schemes provided by the present application will be further described in detail below with reference to the examples, but these examples should not be understood as a limitation to the protection scope of the present application.

### Example 1

This example provided a method for preparing a restorative material for improving inner surface adhesion of dental zirconia, and the preparation method was performed by the following steps:
(1) Components in a ceramic material were mixed uniformly in proportion (referring to Table 1 for the proportion of each component); the mixed raw materials were placed in a crucible, melted at 1,350°C for 10 h, and then quenched with water, obtaining a glass slag.
(2) The glass slag obtained in step (1) was held at 480°C for 180 min, obtaining a glass slag with nucleation; the temperature continued to be raised to 620°C, and the glass slag with nucleation was held at 620°C for 100 min, obtaining lithium metasilicate crystal that was extremely easy to process and grind.
(3) The lithium metasilicate crystal obtained in step (2) was ground, obtaining a glass powder with a particle size of 2 µm, and then a colorant TiO₂ and water were added thereto in sequence and mixed, obtaining a slurry.
(4) The slurry obtained in step (3) was sprayed on the inner surface of a zirconia substrate with a spraying thickness of 30 µm; the sprayed zirconia substrate was subjected to a heat treatment at 850°C, obtaining a lithium disilicate ceramic layer; the lithium disilicate infiltrated into a surface of the zirconia substrate at an adhesion interface between the ceramic layer and the zirconia substrate.

The restorative material prepared above was pasted on an abutment tooth, and the pasting method was performed by the following steps:

The restorative material prepared above was acid-etched with 10% HF for 60 s to fully dissolve the glass substrate in the restorative material, forming a rough zirconia surface, and then the acid-etched restorative material was pasted to an abutment tooth using an adhesive.

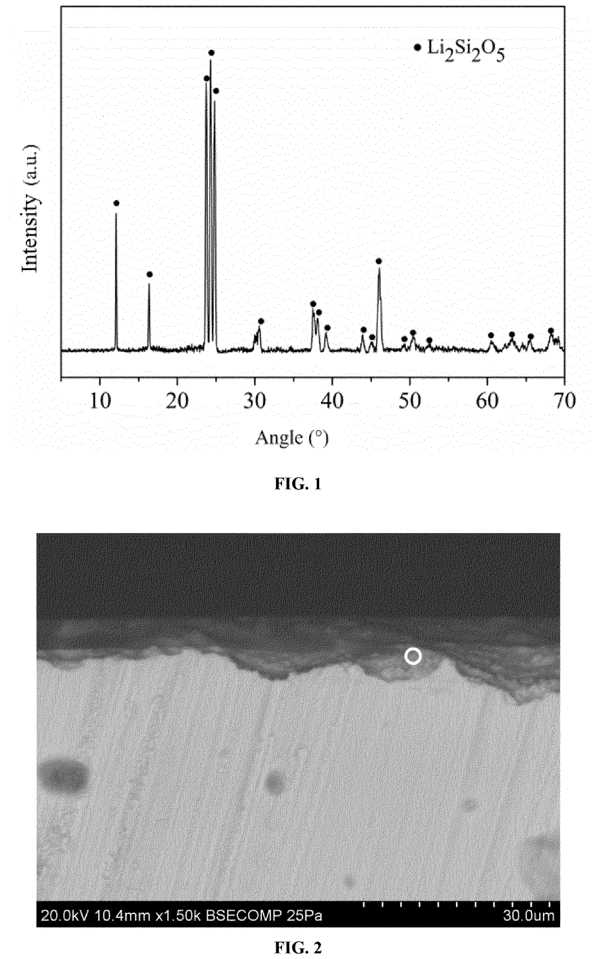

The ceramic layer prepared above was characterized. An XRD pattern thereof was shown in FIG. 1. It can be seen from FIG. 1 that the crystal phase of the ceramic layer is a lithium disilicate crystal. A backscattered electron diagram of the adhesion interface between the ceramic layer and the zirconia substrate was shown in FIG. 2. It can be seen from FIG. 2 that the infiltration occurs at the adhesion interface between the zirconia substrate and the ceramic layer. Table 2 shows the distribution of elements (EDS) at the interface between the restorative material and the zirconia substrate. The EDS data provided in Table 2 also further proves that the lithium disilicate crystals infiltrate into the surface of the zirconia substrate.

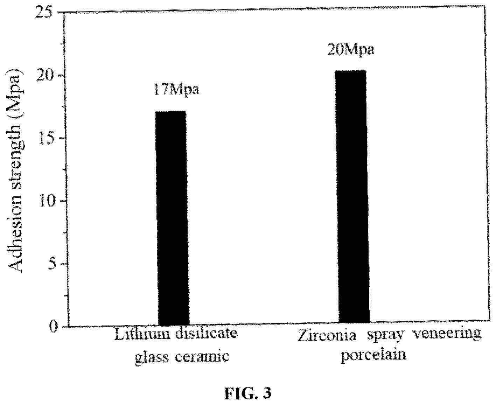

In order to verify the adhesion strength between the restorative material and the adhesive provided in this example, in the present application, a corresponding comparative example was set to test the adhesion strength between a traditional lithium disilicate glass ceramic and an adhesive, and the adhesion strength between the restorative material and the adhesive provided in Example 1. The test results were shown in FIG. 3. It can be seen from FIG. 3 that after the acid etching and the adhesion treatment of the restorative material, the adhesion strength could reach 20 MPa, while the adhesion strength between the traditional lithium disilicate glass ceramic and the adhesive could only reach 17 MPa.

**Table 2 Element distribution at the interface between the restorative material and the zirconia substrate**

| Element | Weight % | Atom % | Error % |
|---|---|---|---|
| C | 21.21 | 32.54 | 11.57 |
| O | 44.05 | 50.73 | 9.25 |
| Na | 3.40 | 2.73 | 8.87 |
| Al | 1.55 | 1.06 | 6.49 |
| Rb | 5.59 | 1.21 | 2.66 |
| Si | 14.91 | 9.78 | 3.76 |
| Y | 1.49 | 0.31 | 10.24 |
| Zr | 7.49 | 1.51 | 3.34 |

### Example 2

This example provided a method for preparing a restorative material for improving inner surface adhesion of dental zirconia, and the preparation method was performed by the following steps:
(1) Components in a ceramic material were mixed uniformly in proportion (referring to Table 1 for the proportion of each component); the mixed raw materials were placed in a crucible, melted at 1,320°C for 12 h, and then quenched with water, obtaining a glass slag.
(2) The glass slag obtained in step (1) was held at 500°C for 160 min, obtaining a glass slag with nucleation; the temperature continued to be raised to 640°C, and the glass slag with nucleation was held at 640°C for 140 min, obtaining lithium metasilicate crystal that was extremely easy to process and grind.
(3) The lithium metasilicate crystal obtained in step (2) was ground, obtaining a glass powder with a particle size of 5 µm, and then a pigment zirconium praseodymium yellow and alcohol were added thereto in sequence and mixed, obtaining a slurry.
(4) The slurry obtained in step (3) was sprayed on the inner surface of a zirconia substrate with a spraying thickness of 60 µm; the sprayed zirconia substrate was subjected to a heat treatment at 880°C, obtaining a lithium disilicate ceramic layer; the lithium disilicate infiltrated into a surface of the zirconia substrate at an adhesion interface between the ceramic layer and the zirconia substrate.

The restorative material prepared above was pasted on an abutment tooth, and the pasting method was performed by the following steps:

The restorative material prepared above was acid-etched with 10% HF for 60 s to fully dissolve the glass substrate in the restorative material, forming a rough zirconia surface, and then the acid-etched restorative material was pasted to an abutment tooth using an adhesive.

### Example 3

This example provided a method for preparing a restorative material for improving inner surface adhesion of dental zirconia, and the preparation method was performed by the following steps:
(1) Components in a ceramic material were mixed uniformly in proportion (referring to Table 1 for the proportion of each component); the mixed raw materials were placed in a crucible, melted at 1,280°C for 12 h, and then quenched with water, obtaining a glass slag.
(2) The glass slag obtained in step (1) was held at 520°C for 120 min, obtaining a glass slag with nucleation; the temperature continued to be raised to 620°C, and the glass slag with nucleation was held at 670°C for 200 min, obtaining lithium metasilicate crystal that was extremely easy to process and grind.
(3) The lithium metasilicate crystal obtained in step (2) was ground, obtaining a glass powder with a particle size of 4 µm, and then a pigment zirconium praseodymium yellow and alcohol were added thereto in sequence and mixed, obtaining a slurry.
(4) The slurry obtained in step (3) was sprayed on the inner surface of a zirconia substrate with a spraying thickness of 100 µm; the sprayed zirconia substrate was subjected to a heat treatment at 900°C, obtaining a lithium disilicate ceramic layer; the lithium disilicate infiltrated into a surface of the zirconia substrate at an adhesion interface between the ceramic layer and the zirconia substrate.

The restorative material prepared above was pasted on an abutment tooth, and the pasting method was performed by the following steps:

The restorative material prepared above was acid-etched with 10% HF for 60 s to fully dissolve the glass substrate in the restorative material, forming a rough zirconia surface, and then the acid-etched restorative material was pasted to an abutment tooth using an adhesive.

### Example 4

This example provided a method for preparing a restorative material for improving inner surface adhesion of dental zirconia, and the preparation method was performed by the following steps:
(1) Components in a ceramic material were mixed uniformly in proportion (referring to Table 1 for the proportion of each component); the mixed raw materials were placed in a crucible, melted at 1,320°C for 10 h, and then quenched with water, obtaining a glass slag.
(2) The glass slag obtained in step (1) was held at 580°C for 60 min, obtaining a glass slag with nucleation; the temperature continued to be raised to 620°C, and the glass slag with nucleation was held at 720°C for 40 min, obtaining lithium metasilicate crystal that was extremely easy to process and grind.
(3) The lithium metasilicate crystal obtained in step (2) was ground, obtaining a glass powder with a particle size of 6 µm, and then a pigment zirconium praseodymium yellow and water were added thereto in sequence and mixed, obtaining a slurry.
(4) The slurry obtained in step (3) was sprayed on the inner surface of a zirconia substrate with a spraying thickness of 80 µm; the sprayed zirconia substrate was subjected to a heat treatment at 820°C, obtaining a lithium disilicate ceramic layer; the lithium disilicate infiltrated into a surface of the zirconia substrate at an adhesion interface between the ceramic layer and the zirconia substrate.

The restorative material prepared above was pasted on an abutment tooth, and the pasting method was performed by the following steps:

The restorative material prepared above was acid-etched with 10% HF for 60 s to fully dissolve the glass substrate in the restorative material, forming a rough zirconia surface, and then the acid-etched restorative material was pasted to an abutment tooth using an adhesive.

### Example 5

This example provided a method for preparing a restorative material for improving inner surface adhesion of dental zirconia. This example differed from Example 1 in that: the mass fraction of GeO2 in the ceramic material was adjusted from 5 wt% to 0.5 wt%, and other components were proportionally increased to compensate for the reduced 4.5 wt% mass fraction of GeO₂ (referring to Table 1 for details); while other process parameters and operation steps were the same as those in Example 1.

### Example 6

This example provided a method for preparing a restorative material for improving inner surface adhesion of dental zirconia. This example differed from Example 1 in that: the mass fraction of GeO₂ in the ceramic material was adjusted from 5 wt% to 12 wt%, and other components were proportionally increased to compensate for the increased 7 wt% mass fraction of GeO₂ (referring to Table 1 for details); while other process parameters and operation steps were the same as those in Example 1.

### Example 7

This example provided a method for preparing a restorative material for improving inner surface adhesion of dental zirconia. This example differed from Example 1 in that: in step (4), the heat treatment was conducted at 800°C; while other process parameters and operation steps were the same as those in Example 1.

### Example 8

This example provided a method for preparing a restorative material for improving inner surface adhesion of dental zirconia. This example differed from Example 1 in that: in step (4), the heat treatment was conducted at 920°C; while other process parameters and operation steps were the same as those in Example 1.

**Table 1 Raw material proportions in Examples 1-6**

| | Example 1 | Example 2 | Example 3 | Example 4 | Example 5 | Example 6 |
|---|---|---|---|---|---|---|
| SiO₂ | 56 wt% | 60 wt% | 62 wt% | 58 wt% | 58.65 wt% | 51.87 wt% |
| B₂O₃ | 3 wt% | 4 wt% | 2 wt% | 3 wt% | 3.14 wt% | 2.78 wt% |
| GeO₂ | 5 wt% | 5 wt% | 4 wt% | 6 wt% | 0.5 wt% | 12 wt% |
| Al₂O₃ | 2 wt% | 1 wt% | 0.5 wt% | 1 wt% | 2.09 wt% | 1.85 wt% |
| Li₂O | 22 wt% | 23 wt% | 24 wt% | 20 wt% | 23.04 wt% | 20.38 wt% |
| Nb₂O₅ | 5 wt% | 2 wt% | 3 wt% | 4 wt% | 5.24 wt% | 4.63 wt% |
| Na₂O | 3 wt% | 3 wt% | 2 wt% | 3 wt% | 3.14 wt% | 2.78 wt% |
| ZrO₂ | 1 wt% | 0.5 wt% | 0.5 wt% | 1 wt% | 1.05 wt% | 0.93 wt% |
| P₂O₅ | 2 wt% | 1 wt% | 1 wt% | 3 wt% | 2.09 wt% | 1.85 wt% |
| La₂O₃ | 0.5 wt% | 0.5 wt% | - | 1 wt% | 0.52 wt% | 0.46 wt% |
| Tb₂O₃ | 0.5 wt% | - | 1 wt% | - | 0.54 wt% | 0.47 wt% |

The restorative materials prepared in Examples 1-8 were tested, and the test method was performed by the following steps:
(1) Spray thickness: the spray thickness in the present application was analyzed according to test requirements in Section 7.5 of the YY1042-2003industry standard.
(2) Chemical solubility: the chemical solubility in the present application was tested and analyzed according to the ISO6872:2008 international standard.
(3) Thermal expansion coefficient: the thermal expansion coefficient in the present application was tested and analyzed according to the ISO6872:2008 international standard.
(4) Adhesion strength: the adhesion strength in the present application was based on the data test according to the YY/T0518-2009 industry standard.

The test results were shown in Table 3:

**Table 3 Performance data of restorative materials prepared in Examples 1-8**

| | Chemical solubility (µg/cm²) | Thermal expansion coefficient (10⁻⁶/K) | Adhesion strength (MPa) |
|---|---|---|---|
| Example 1 | 10 | 9.7 | 20 |
| Example 2 | 18 | 100 | 18 |
| Example 3 | 23 | 9.9 | 21 |
| Example 4 | 14 | 10.2 | 22 |
| Example 5 | 20 | 11.7 | 6 |
| Example 6 | 40 | 11.5 | 5 |
| Example 7 | 15 | 10.4 | 8 |
| Example 8 | 20 | 120 | 4 |

It can be seen from the data in Table 3 that:
(1) It can be seen from Example 1 that after spraying the slurry on the surface of zirconia and performing the heat treatment, the crystal phase of the prepared restorative material is Li₂Si₂O₅. Since the restorative material has been crystallized to form glass-ceramics, the sample has a chemical solubility of 10 µg/cm², meeting the requirements of the ISO6872:2008 international standard. The restorative material has a thermal expansion coefficient of 9.7×10⁻⁶/K, which is not more than 1.0×10⁻⁶/K different from the thermal expansion coefficient of normal zirconia at 10.5×10⁻⁶/K. Therefore, the relatively-matched thermal expansion coefficients of the two could well ensure that the restorative material may not crack or peel off during use. In addition, zirconia treated with the restorative material has an adhesion strength of 20 Mpa, while the lithium disilicate-based glass ceramic has an adhesion strength of 17 Mpa through a comparative study, indicating that the restorative material has significantly improved the adhesion of zirconia.
(2) It can be seen from Example 2 that after spraying the slurry on the surface of zirconia and performing the heat treatment, the crystal phase of the prepared restorative material is Li₂Si₂O₅. Since the restorative material has been crystallized to form glass-ceramics, the sample has a chemical solubility of 18 µg/cm², meeting the requirements of the ISO6872:2008 international standard. The restorative material has a thermal expansion coefficient of 10.0×10⁻⁶/K, which is not more than 1.0×10⁻⁶/K different from the thermal expansion coefficient of normal zirconia at 10.5×10⁻⁶/K. Therefore, the relatively-matched thermal expansion coefficients of the two could well ensure that the restorative material may not crack or peel off during use. In addition, zirconia treated with the restorative material has an adhesion strength of 18 Mpa, while the lithium disilicate-based glass ceramic has an adhesion strength of 17 Mpa through a comparative study, indicating that the restorative material has significantly improved the adhesion of zirconia.
(3) It can be seen from Example 3 that after spraying the slurry on the surface of zirconia and performing the heat treatment, the crystal phase of the prepared restorative material is Li₂Si₂O₅. Since the restorative material has been crystallized to form glass-ceramics, the sample has a chemical solubility of 23 µg/cm², meeting the requirements of the ISO6872:2008 international standard. The restorative material has a thermal expansion coefficient of 9.9×10⁻⁶/K, which is not more than 1.0×10⁻⁶/K different from the thermal expansion coefficient of normal zirconia at 10.5×10⁻⁶/K. Therefore, the relatively-matched thermal expansion coefficients of the two could well ensure that the restorative material may not crack or peel off during use. In addition, zirconia treated with the restorative material has an adhesion strength of 21 Mpa, while the lithium disilicate-based glass ceramic has an adhesion strength of 17 Mpa through a comparative study, indicating that the restorative material has significantly improved the adhesion of zirconia.
(4) It can be seen from Example 4 that after spraying the slurry on the surface of zirconia and performing the heat treatment, the crystal phase of the prepared restorative material is Li₂Si₂O₅. Since the restorative material has been crystallized to form glass ceramics, the sample has a chemical solubility of 14 µg/cm², meeting the requirements of the ISO6872:2008 international standard. The restorative material has a thermal expansion coefficient of 10.2×10⁻⁶/K, which is not more than 1.0×10⁻⁶/K different from the thermal expansion coefficient of normal zirconia at 10.5×10⁻⁶/K. Therefore, the relatively-matched thermal expansion coefficients of the two could well ensure that the restorative material may not crack or peel off during use. In addition, zirconia treated with the restorative material has an adhesion strength of 22 Mpa, while the lithium disilicate-based glass ceramic has an adhesion strength of 17 Mpa through a comparative study, indicating that the restorative material has significantly improved the adhesion of zirconia.
(5) It can be seen from Examples 5 and 6 that too high or too low mass fraction of GeO₂ may affect the adhesion strength of the final restorative material. This is because when the content of GeO₂ is low, the amount of GeO₂ to substitute SiO₂ in the glass-ceramics will be reduced, resulting in the uneven melting of glass-ceramics below 1,350°C and serious phase separation, thereby leading to a decrease in adhesion strength of the restorations; when the content of GeO₂ is too high, the ratio of Si to Li will be significantly reduced, resulting in the lithium disilicate crystal formed by crystallization being very small, and the crystals cannot achieve effective cross-linking and interlocking so that the adhesion strength of the restorations cannot be improved.
(6) It can be seen from Examples 7 and 8 that too high or too low heat treatment temperature may affect the adhesion strength of the final restorative material. This is because when the heat treatment temperature is too low, it is difficult for the lithium metasilicate crystals in the slurry to fully react to form lithium disilicate crystals, and there are still a large number of lithium metasilicate crystals that are easy to be etched by HF. As a result, during the acid etching, it is easy to leave too many pits, leading to the extremely easy formation of pores between the adhesion agent and the pits, further resulting in a decreased final adhesion strength; when the heat treatment temperature is too high, the crystallized lithium disilicate crystals may be re-melted to form glass, failing to achieve a high adhesion strength with an adhesive.

Based on the above examples, it can be seen that the restorative material of the present application can effectively solve the problem that zirconia is difficult to be bound by adding GeO₂, optimizing a composition ratio and regulating a heat treatment process. Moreover, the difference between the thermal expansion coefficient and the zirconia can be stably maintained within 1.0×10⁻⁶/K, thus well ensuring thermal matching between the restorative material and the zirconia. In addition, after corresponding heat treatment, acid etching and adhesion agent treatment, the adhesion strength of zirconia can be greater than or equal to 18 Mpa.

The above described are merely specific implementations of the present application, and the protection scope of the present application is not limited thereto. Any modification or replacement easily conceived by those skilled in the art within the technical scope of the present application should fall within the protection scope of the present application.

## Claims

1. A ceramic material for improving surface adhesion of dental zirconia, **characterized by** comprising SiO₂, B₂O₃, GeO₂, Al₂O₃, Li₂O, Nb₂O₅, Na₂O, ZrO₂, P₂O₅, and a rare earth oxide.

2. The ceramic material according to claim 1, wherein based on a mass fraction of the ceramic material as 100 wt%, the ceramic material comprises following components by mass fraction:
| | |
|---|---|
| SiO₂ | 54-68 wt% |
| B₂O₃ | 2-6 wt% |
| GeO₂ | 1-10 wt% |
| Al₂O₃ | 0.2-4 wt% |
| Li₂O | 20-40 wt% |
| Nb₂O₅ | 2-10 wt% |
| Na₂O | 2-10 wt% |
| ZrO₂ | 0.2-5 wt% |
| P₂O₅ | 0.2-8 wt% |
| the rare earth oxide | 0-4 wt% |

3. The ceramic material according to claim 1, wherein based on a mass fraction of the ceramic material as 100 wt%, the ceramic material comprises following components by mass fraction:
| | |
|---|---|
| SiO₂ | 55-65 wt% |
| B₂O₃ | 3-5 wt% |
| GeO₂ | 2-6 wt% |
| Al₂O₃ | 0.8-3 wt% |
| Li₂O | 20-40 wt% |
| Nb₂O₅ | 3-8 wt% |
| Na₂O | 3-6 wt% |
| ZrO₂ | 0.2-3 wt% |
| P₂O₅ | 0.4-6 wt% |
| the rare earth oxide | 0.4-3 wt% |

4. The ceramic material according to any one of claims 1 to 3, wherein the rare earth oxide comprises any one or a combination of at least two selected from the group consisting of Nd₂O₃, Tb₂O₃, Pr₆O₁₁, La₂O₃, Eu₂O₃, and Er₂O₃.

5. A restorative material for improving surface adhesion of dental zirconia, **characterized by** comprising a zirconia substrate and a ceramic layer on a surface of the zirconia substrate, wherein at an interface between the ceramic layer and the zirconia substrate, lithium disilicate in the ceramic layer infiltrates into the surface of the zirconia substrate; and
the ceramic layer is prepared by the ceramic material according to any one of claims 1 to 4.

6. The restorative material according to claim 5, wherein the zirconia substrate comprises any one selected from the group consisting of dental veneers, inlays, onlays, abutment teeth, single crowns, anterior multi-unit pontic and posterior multi-unit pontica.

7. A method for preparing the restorative material according to claim 5 or 6, **characterized by** comprising:
(I) mixing components in a ceramic material in proportion, followed by melting, quenching with water to obtain a glass slag, and subjecting the glass slag to a nucleation heat treatment and a crystallization heat treatment in sequence to obtain a lithium metasilicate glass ceramic;
(II) grinding the lithium metasilicate glass ceramic to obtain a glass powder and mixing the glass powder, a coloring material and a solvent to obtain a slurry; and
(III) spraying the slurry on an inner surface of a zirconia substrate and conducting a heat treatment so that lithium metasilicate crystals in the slurry are transformed into a lithium disilicate main crystalline phase and infiltrate into a surface of the zirconia substrate.

8. The preparation method according to claim 7, wherein in step (I), the melting is conducted in a crucible;
the melting is conducted at a temperature within a range of 1,050°C-1,350°C; and
the melting is conducted for 1 h to 12 h.

9. The preparation method according to claim 7 or 8, wherein the nucleation heat treatment is conducted at a temperature within a range of 460-580°C; and
the nucleation heat treatment is conducted for 30-300 min.

10. The preparation method according to claim 7 or 8, wherein the crystallization heat treatment is conducted at a temperature within a range of 600-780°C, and the crystallization heat treatment is conducted for 30-300 min.

11. The preparation method according to claim 7, wherein in step (II), the glass powder has a particle size of 0.5-20 µm.

12. The preparation method according to claim 7, wherein the coloring material comprises a colorant and/or a pigment; the colorant comprises any one or a combination of at least two selected from the group consisting of TiO₂, CeO₂, CuO, Cr₂O₃, MnO₂, SeO₂, V₂O₅, Pr₆O₁₁, Tb₄O₇, In₂O₃, and TaO₂; and the pigment comprises any one or a combination of at least two selected from the group consisting of zirconium praseodymium yellow, zirconium iron red, zirconium vanadium blue, and nickel black.

13. The preparation method according to claim 7, wherein in step (III), the slurry has a spraying thickness of 5 µm to 200 µm.

14. The preparation method according to claim 7, wherein in step (III), the heat treatment is conducted at a temperature within a range of 820-900°C.

15. The preparation method according to claim 15, wherein the heat treatment is conducted at the temperature within the range of 850-890°C.

16. A method for pasting the restorative material according to claim 5 or 6 or a restorative material prepared by the preparation method according to any one of claims 7 to 15, comprising steps of
acid etching the restorative material to form an acid-etched surface, coating the acid-etched surface with an adhesive, and pasting the restorative material on a surface of an abutment tooth through the adhesive.
